# EUROPEAN PATENT APPLICATION

(11) **EP 4 382 104 A1**
(43) Date of publication of application: **12.06.2024**
(21) Application number: 22306788.5
(22) Date of filing: 05.12.2022
(51) Int. Cl.: A61K 31/4453, A61K 9/00, A61P 17/00

(54) **DYCLONINE FOR USE IN THE TOPICAL TREATMENT OF HAND-FOOT SYNDROME**

(71) Applicant: Assistance Publique Hôpitaux de Paris, 75012 Paris (FR)
(72) Inventor: GRECO, Céline, 75012 PARIS (FR)
(74) Representative: Gevers & Orès

(57) **Abstract**

The present invention relates to a compound selected from dyclonine, a dyclonine analog and a pharmaceutically acceptable salt thereof, or a composition comprising such a compound, for use in the topical treatment and prevention of hand-foot syndrome.

## Description

The present invention relates to a compound selected from dyclonine, a dyclonine analog and a pharmaceutically acceptable salt thereof, or a composition comprising such a compound, for use in the topical treatment and prevention of hand-foot syndrome.

Hand-foot syndrome is a side effect of some cancer treatment. Hand-foot syndrome causes redness, swelling, pain and burning sensations on the palms of the hands and/or the soles of the feet. In severe cases, patients may notice dryness, cracking or peeling skin, blisters, ulcers, hyperkeratosis or sores appearing on the skin, intense pain, difficulty walking or using the hands. Hand-foot syndrome sometimes happens elsewhere on the skin, such as the knees or elbows but this is less common. Not everyone who received a cancer treatment develops hand-food syndrome and the severity of hand-foot syndrome can be different for everyone. Hand-foot syndrome tends to appear within the first two to three months of treatment.

Hand-foot syndrome treatment is largely based on symptom control. Moisturizers and emollient creams can be used to keep skin hydrated. Corticosteroid creams can also be used in case of swelling or inflammation. However, in severe cases, the dose of chemotherapy must be adjusted and reduced to manage this side effect or the cancer treatment must be postponed until symptoms improve.

There thus remains a genuine need for new treatments of hand-foot syndrome to improve patient care and facilitate the process of cancer therapies.

The present invention is believed to meet such need by providing a topical composition for treating or preventing hand-foot syndrome.

Dyclonine is a chemical compound of formula (I):

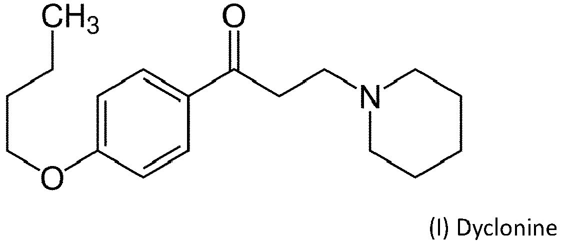

Dyclonine is an oral anesthetic used to provide topical anesthesia to mucous membranes through sodium channel inhibition. It is the active ingredient in over-the-counter throat lozenges. It has been used as a local anesthetic agent prior to laryngoscopy, bronchoscopy, esophagoscopy, or endotracheal intubation. Recently, additional activities of dyclonine have been discovered. Dyclonine represents a novel therapeutic strategy that can potentially be repurposed for the treatment of Friedreich's ataxia. Dyclonine enhances the cytotoxic effect of proteasome inhibitors on cancer and multiple myeloma cells.

The Inventors have in a surprising manner shown that the topical administration of dyclonine reduces the pain and the damages of the skin recovery of the skin for patients suffering from hand-foot syndrome. Moreover, since dyclonine is not administered by a systemic route, there are few or no side effects.

Thus, the present invention concerns a compound selected from dyclonine, a dyclonine analog and a pharmaceutically acceptable salt thereof, for use in the topical treatment or prevention of hand-foot syndrome.

The present invention further relates to a topical composition comprising a compound according to the invention as active ingredient and at least one pharmaceutically acceptable excipient, for use in the treatment or prevention of hand-foot syndrome.

The present invention further relates to a method for treating and/or preventing hand-foot syndrome by means of topical administration, to a patient in need thereof, of an effective amount of a compound or composition according to the invention.

The present invention further relates to the use of a compound or composition comprising a compound according to the invention for the manufacture of a topical medication for treating and/or preventing hand-foot syndrome.

"Dyclonine" (or "dyclocaine") as used herein refers to the molecule 1-(4-Butoxyphenyl)-3-(1-piperidinyl)-1-propanone, polymorphs, salts, solvates or hydrates thereof.

"Dyclonine analog", as used herein, refers to a chemical compound having biological activities similar to those of dyclonine but with a slightly altered chemical structure.

In the present invention, "pharmaceutically acceptable" is intended to mean that which is useful in the preparation of a pharmaceutical composition, generally safe, nontoxic and neither biologically nor otherwise undesirable, and acceptable for both veterinary and human pharmaceutical use.

"Pharmaceutically acceptable salt" of a compound is intended to mean a salt that is pharmaceutically acceptable, as defined herein, and that has the desired pharmacological activity of the parent compound.

Salts of dyclonine include salts of acidic or basic groups present in compounds of the invention. Pharmaceutically acceptable salts of dyclonine include, but are not limited to, dyclonine hydrochloride.

In the present invention, the term "hand-foot syndrome" (also called "palmo-plantar erythrodysesthesia") refers to a skin reaction on the palms of the hands and/or the soles of the feet as a result of certain cancer treatments. Grading of hand-foot syndrome according to the CTCAE (Common Terminology Criteria for Adverse Events) is as follows:
Grade 1: Minimal skin changes or dermatitis (e.g., erythema, oedema, or hyperkeratosis) without pain.
Grade 2: Skin changes (e.g., peeling, blisters, bleeding, fissures, edema, or hyperkeratosis) with pain; limiting instrumental activities of daily living) .
Grade 3: Severe skin changes (e.g., peeling, blisters, bleeding, fissures, edema, or hyperkeratosis) with pain; limiting self-care activities of daily living).

In an embodiment, the hand-foot syndrome is caused by a chemotherapy.

In the present invention, "chemotherapy" refers to the treatment of cancer by the administration of a chemical substance to the patient. Chemotherapies that are more likely to cause hand-foot syndrome include - but are not limited to: Doxorubicin, liposomal Doxorubicin, Idarubicin, Daunorubicin, 5-fluorouracil, Ixabepilone, Capecitabine, Docetaxel, Paclitaxel, Cytarabine, Fludarabine, Bleomycin, Cisplatin, Cyclophosphamide, Doxifluridine, Etoposide, Gemcitabine, Hydroxyurea, Methotrexate, Mitotane, Tegafur, Thiotepa, Vemurafenib and Vinorelbine.

In an embodiment, the hand-foot syndrome is caused by a targeted therapy.

In the present invention, "targeted therapy" refers to the treatment of cancer by the administration of a chemical substance or a drug that precisely attacks certain types of cancer cells. Targeted therapies that are more likely to cause hand-foot syndrome include - but are not limited to: Axitinib, Cabozantinib, Regoferenib, Sorafenib, Sunitinib, Margetuximab, Pazopanib and Lapatinib.

An anti-cancer therapy that can cause hand-foot syndrome can comprise more than one active substance.

In an embodiment, the hand-foot syndrome is caused by a combination of drugs.
In an embodiment, the hand-foot syndrome is caused by a combination of folinic acid, 5-fluorouracil, irinotecan and oxaliplatin (combination called "Folfirinox").

By "treatment" is meant, according to the present invention, the inhibition of the development, preferably the regression or more preferably the disappearance of the skin reactions and/or pain and/or limitations of activities of daily living associated to hand-foot syndrome.

By "prevention" is meant, according to the present invention, to prevent or delay the appearance of the skin reactions and/or pain and/or limitations of activities of daily living associated to hand-foot syndrome.

The treatment or prevention according to the invention applies to humans or animals, preferably to humans.

By "topical" is meant, according to the present invention, that the compound or composition according to the invention will be administered by application on the skin surface or the mucous membranes.

The topical composition according to the invention may in particular be in any form allowing topical application, such as a cream, a gel, an ointment, a solution, a lotion, a spray, an aerosol spray, an aerosol foam or a patch. Preferably, the composition according to the invention will be in cream or gel form.

In an embodiment, the topical composition of the invention can be applied to the skin by an applicator, such as a roll-on, a stick, an impregnated wipe or an impregnated glove.

In an embodiment, the composition of the invention can also be dispensed from a pump pack or from an aerosol container.

In an embodiment, the topical composition according to the invention may be in the form of a thermoreversible spray. Due to its thermoreversible nature, a cold formulation can be sprayed as a liquid and then transition to a gel at skin temperatures.

In an embodiment, the topical composition of the invention comprises a compound according to the invention in a concentration from 0.1 to 30%, in particular from 1% to 20%, more particularly from 4% to 10%, by weight relative to the weight of the composition.

In an embodiment, the topical composition of the invention comprises a compound according to the invention in a concentration of at least 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19% , 20%, 21%, 22%, 23%, 24%, 25%, 26%, 27%, 28%, 29% or 30% by weight relative to the weight of the composition.
In an embodiment, the topical composition of the invention comprises a compound according to the invention in a concentration of 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19% , 20%, 21%, 22%, 23%, 24%, 25%, 26%, 27%, 28%, 29% or 30% by weight relative to the weight of the composition.

In an embodiment, the topical composition of the invention comprises a compound according to the invention in a concentration of 1% by weight relative to the weight of the composition.

In an embodiment, the topical composition of the invention comprises a compound according to the invention in a concentration of 4% by weight relative to the weight of the composition.

In an embodiment, the topical composition of the invention comprises a compound according to the invention in a concentration of 5% by weight relative to the weight of the composition.

In an embodiment, the topical composition of the invention comprises a compound according to the invention in a concentration of 10% by weight relative to the weight of the composition.

In an embodiment, the topical composition of the invention is formulated into unit dose form.

The compound ortopical composition according to the invention will be administered one or more times per day, the duration being variable according to the severity of the symptoms and easily adjustable by the person skilled in the art or the practitioner.

In an embodiment, the topical composition of the invention is applied to the affected area once daily, twice daily, three times daily, once every second day, three times weekly, twice weekly or once weekly.

By "pharmaceutically acceptable excipient" is meant, according to the invention, an excipient that is compatible with the other ingredients of the composition and that produces no adverse effect, allergic reaction or other undesirable reaction when it is administered to a human or an animal.

According to the invention, by "excipient" is meant in particular one or more surfactants, for example macrogols, hydroxy stearates, ethoxylated fatty acid esters, ethoxylated fatty alcohols; one or more solvents, such as for example octyldodecanol, propylene glycol dicaprylocaprate; one or more hydrosoluble polymers, for example PVP, hyaluronic acid or sodium hyaluronate; one or more thickeners such as for example natural or semisynthetic gums; one or more gelling agents, for example carbomers; one or more inorganic fillers, for example zinc oxide, talc, clays; one or more emulsifiers such as cetearyl alcohol; one or more preservatives, for example phenoxyethanol; one or more antibacterials; one or more antiseptics; one or more antioxidants, for example tocopherol acetate; one or more chelating agents, for example EDTA; one or more pigments; one or more fragrances; one or more colorants; one or more pH adjustors such as salts, acids, bases; or a mixture thereof.

In an embodiment, the topical composition of the invention further comprises a penetration enhancer.

By "penetration enhancer" is meant, according to the invention, a chemical substance that promotes the transdermal penetration of the drug applied at the surface of the skin. For example, a penetration enhancer can be selected among alcohols, amides, esters, glycols, fatty acids, pyrrolidones, sulfoxides, surfactants, terpenes, urea, cyclodextrins, water, vitamin E or phospholipids.

In an embodiment, the topical composition further comprises a base allowing transdermal diffusion of said compound. In particular, such a base may be selected from the group comprising Excipial Hydrocrème^{®}, Codexial Obase^{®}, Pentravan^{®}, Pentravan^{®} Plus, Phytobase^{®}, Lipovan^{®} and Pluronic Lecithin Organogel (PLO), preferably Pentravan^{®}, preferably Excipial Hydrocrème^{®} or Codexial Obase^{®}.

In an embodiment, the topical composition of the invention comprises dyclonine in a concentration from 0.1 to 30%, preferably in a concentration from 0.5% to 20%, more preferably in a concentration from 1% to 10%, by weight relative to the weight of the composition in a base of Excipial Hydrocrème^{®}.

In an embodiment, the topical composition of the invention comprises dyclonine in a concentration from 0.1 to 30%, preferably in a concentration of from 0.5% to 20%, more preferably in a concentration from 1% to 10%, by weight relative to the weight of the composition in a base of Codexial Obase^{®}.

The following Examples are put forth so as to provide those of ordinary skill in the art with a complete disclosure and description of how to make and use the present invention, and are not intended to limit the scope of what the Inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### FIGURE LEGENDS

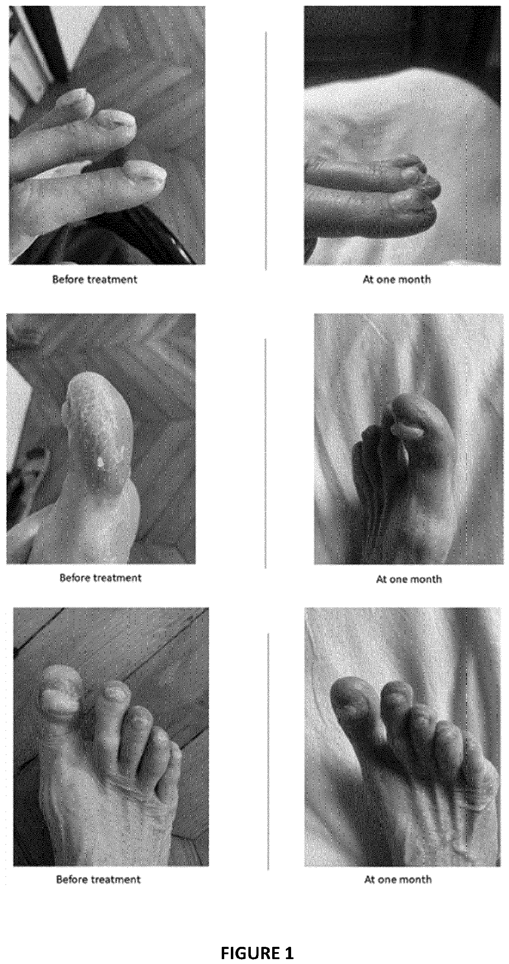

**Figure 1****.** Before and after treatment by dyclonine 4%.

### EXAMPLES

### Example 1

A patient aged 62 suffered from a metastatic pancreatic cancer. She developed a severe and painful palmo-plantar erythrodysesthesia under FOLFIRINOX therapy with painful onycholysis with oozing, paronychia, skin desquamation and neuropathic pain.

She was treated with a cream of dyclonine 4 % applied locally twice a day.

This patient shown an improvement after 1 month of use. We observed a great improvement of the paronychia, the drying of oozing lesions under the nails and the almost complete disappearance of pain (Figure 1).

### Example 2

A patient aged 47 suffered from pancreatic cancer. She developed a severe and painful palmo-plantar erythrodysesthesia under FOLFIRINOX therapy with painful nail detachment, hyperkeratosis, skin desquamation, neuropathic pain.

He was treated with a cream of dyclonine 4 % applied locally twice a day.

This patient shown an improvement after 3 weeks of use with disappearance of hyperkeratosis, improvement of skin desquamation and neuropathic pain.

### Example 3

A patient aged 40 suffered from breast cancer. She developed a painful palmo-plantar erythrodysesthesia under taxane therapy with painful onycholysis (hands and feet), hyperkeratosis and neuropathic pain.

She was treated with a cream of dyclonine 4 % applied locally twice a day.

This patient shown an improvement after 3 weeks of use with almost complete disappearance of hyperkeratosis, improvement of skin desquamation and neuropathic pain.

### Example 4

A patient aged 60 suffered from colon cancer. He developed a painful palmo-plantar erythrodysesthesia under capecitabine therapy with painful onycholysis (hands and feet), hyperkeratosis and neuropathic pain.

He was treated with a cream of dyclonine 4 % applied locally twice a day.

This patient shown an improvement after 1 month of use with almost complete relief of hyperkeratosis and neuropathic pain.

## Claims

1. A compound selected from dyclonine, a dyclonine analog and a pharmaceutically acceptable salt thereof, for use in the topical treatment or prevention of hand-foot syndrome.

2. A topical composition comprising a compound selected from dyclonine, a dyclonine analog and a pharmaceutically acceptable salt thereof, as active ingredient and at least one pharmaceutically acceptable excipient, for use in the treatment or prevention of hand-foot syndrome.

3. The topical composition for use according to claim 2, wherein the hand-foot syndrome is caused by a chemotherapy, preferably selected from the group comprising Doxorubicin, liposomal Doxorubicin, Idarubicin, Daunorubicin, 5-fluorouracil, Ixabepilone, Capecitabine, Docetaxel, Paclitaxel, Cytarabine, Fludarabine, Bleomycin, Cisplatin, Cyclophosphamide, Doxifluridine, Etoposide, Gemcitabine, Hydroxyurea, Methotrexate, Mitotane, Tegafur, Thiotepa, Vemurafenib and Vinorelbine

4. The topical composition for use according to claim 2, wherein the palmar-plantar erythrodysesthesia is caused by a targeted therapy, preferably selected from the group comprising Axitinib, Cabozantinib, Regoferenib, Sorafenib, Sunitinib, Margetuximab, Pazopanib and Lapatinib.

5. The topical composition for use according to claim 2, wherein the hand-foot syndrome is caused by a combination of drugs.

6. The topical composition for use according to claim 5, wherein the hand-foot syndrome is caused by a combination of folinic acid, 5-fluorouracil, irinotecan and oxaliplatin.

7. The topical composition for use according to any one of claims 2-6, wherein the compound is in a concentration from 0.1 to 30%, in particular from 1% to 20%, more particularly from 4% to 10%, by weight relative to the weight of the composition.

8. The topical composition for use according to any one of claims 2-7, wherein the topical composition is formulated into unit dose form.

9. The topical composition for use according to any one of claims 2-8, wherein the topical composition is applied to the affected area once daily, twice daily, three times daily, once every second day, three times weekly, twice weekly or once weekly.

10. The topical composition for use according to any one of claims 2-9, further comprising a penetration enhancer.
